# FASCICULE DE BREVET EUROPEEN

(11) **EP 3 719 426 B1**
(45) Date de publication et mention de la délivrance du brevet: **05.06.2024**
(21) Numéro de dépôt: 20165755.8
(22) Date de dépôt: 26.03.2020
(51) Int. Cl.: F25J 1/00, F25J 3/02, F25J 3/06, F25J 1/02, C10L 3/10

(54) **PURIFICATION ET LIQUÉFACTION DU BIOGAZ PAR COMBINAISON D'UN SYSTÈME DE CRISTALLISATION AVEC UN ÉCHANGEUR DE LIQUÉFACTION**
REINIGUNG UND VERFLÜSSIGUNG VON BIOGAS DURCH KOMBINATION EINES KRISTALLISIERUNGSSYSTEMS MIT EINEM VERFLÜSSIGUNGSTAUSCHER
BIOGAS PURIFICATION AND LIQUEFACTION BY COMBINING A CRYSTALLISATION SYSTEM WITH A LIQUEFACTION HEAT EXCHANGER

(30) Priorité: 04.04.2019 FR 1903602
(43) Date de publication de la demande: 07.10.2020
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: TRUEBA, Antonio, 78350 Les Loges-En-Josas (FR); TOVAR RAMOS, Jorge Ernesto, 78350 Les Loges-En-Josas (FR); CRAYSSAC, Frédéric, 78350 Les Loges-En-Josas (FR); VALENTIN, Solène, 38360 Sassenage (FR); ANDRICH, Marine, 78350 Les Loges-En-Josas (FR)
(74) Mandataire: Air Liquide

(56) Documents cités:
- EP-A1- 2 277 614
- WO-A1-2016/179115
- CN-A- 102 628 635
- FR-A1- 3 050 655
- JP-A- 2009 019 192

## Description

La présente invention est relative à une installation et à un procédé de production de méthane liquide à partir d'un flux gazeux d'alimentation comprenant au moins du méthane et du dioxyde de carbone. Elle concerne en particulier une installation et à un procédé de production de méthane liquide à partir de biogaz.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Dans de nombreuses régions en Europe et dans le monde, le réseau de gaz naturel n'est pas toujours accessible à proximité des zones de production de déchets fermentescibles. De plus, s'il n'y a pas de besoin de chaleur sur le lieu de production de biogaz, en fonction du prix d'achat de l'électricité, la cogénération n'a pas toujours un rendement suffisant pour rentabiliser l'investissement important dans une unité de digestion. Il est alors intéressant dans ces 2 cas de transporter le biogaz vers un point de distribution ou de consommation. La liquéfaction du biogaz après épuration permettrait de transporter le biométhane à moindre coût.

Aujourd'hui, les procédés de purification du biogaz sont principalement basés sur les principes de l'absorption, la perméation ou l'adsorption. Ces systèmes nécessitent alors le rajout d'un module supplémentaire pour obtenir le biométhane sous forme liquide. De plus, dans la plupart des cas, le contenu en CO2 dans le biogaz à l'issue de cette étape de purification est toujours trop important pour alimenter de tels systèmes de liquéfaction.

Partant de là, un problème qui se pose est de fournir une installation unique de séparation et de liquéfaction du méthane issu d'un flux gazeux comprenant au moins du méthane et du dioxyde de carbone, le flux gazeux étant de préférence du biogaz. Le but étant d'obtenir du méthane liquide, de préférence du biométhane liquide, pour faciliter son stockage et/ou son transport.

Le document JP 2009 019192 A décrit un procédé et un appareil pour purifier le gaz naturel afin de séparer les hydrocarbures autres que le méthane en mélangeant le gaz naturel avec du gaz naturel liquéfié et en introduisant le mélange sous forme de pulvérisation dans un réservoir de séparation. Néanmoins il ne concerne pas la séparation du CO2 et du méthane et la liquéfaction du méthane dans une seule installation sans nécessiter de modules séparés pour la purification du méthane gazeux et pour la liquéfaction.

Une solution de la présente invention est une installation de production de méthane liquide à partir d'un flux gazeux d'alimentation 1 comprenant au moins du méthane et du dioxyde de carbone comprenant dans le sens de circulation du flux gazeux :
- un système de cristallisation 5 du dioxyde de carbone fonctionnant en continu comprenant à contre-courant du flux gazeux d'alimentation la circulation d'un flux 6 comprenant en majorité du méthane liquide, et permettant la production un flux gazeux enrichi en méthane 7,
- un échangeur de liquéfaction 8 du flux gazeux enrichi en méthane 7 sortant du système de cristallisation, permettant de produire du méthane liquide,
- un moyen permettant de récupérer le méthane liquide en sortie de l'échangeur de liquéfaction, et
- un moyen permettant de renvoyer une partie du méthane liquide vers le système de cristallisation, ladite partie du méthane liquide formant le flux comprenant en majorité du méthane liquide.

Selon le cas, l'installation selon l'invention peut présenter une ou plusieurs des caractéristiques suivantes :
- ladite installation comprend en amont du système de cristallisation un moyen C permettant d'amener le flux gazeux d'alimentation à une température comprise entre - 50°C et -85°C, de préférence entre -57°C et -75°C ;
- le flux gazeux d'alimentation comprend de l'eau et le moyen C comprend au moins deux échangeurs 3a et 3b disposés en parallèle, les échangeurs suivant chacun un cycle comprenant une étape de production et une étape de régénération, avec à chaque instant du cycle un échangeur en étape de production et un échangeur en régénération ;
- l'installation comprend en amont du moyen C un moyen C' 2 permettant d'amener le flux gazeux d'alimentation à une température comprise entre 0°C et 20°C, de préférence comprise entre 0°C et 10°C ;
- l'installation comprend un conduit relié à un fond inférieur du système de cristallisation, et un système de séparation de phases liquide-solide placé sur ce conduit et permettant de récupérer des cristaux de CO2 ;
- le flux d'alimentation est à une pression comprise entre la pression atmosphérique et 20 bar, de préférence entre la pression atmosphérique et 5 bar.

La présente invention a également pour objet un procédé de production de méthane liquide à partir d'un flux gazeux d'alimentation comprenant au moins du méthane et du dioxyde de carbone et mettant en oeuvre une installation telle que définie précédemment, ledit procédé comprenant :
- une étape continue de cristallisation du dioxyde de carbone contenu dans le flux gazeux d'alimentation par injection de celui-ci dans le système de cristallisation du dioxyde de carbone comprenant à contre-courant du flux gazeux d'alimentation la circulation d'un flux comprenant en majorité du méthane liquide ;
- une étape de récupération de méthane gazeux en partie supérieure du système de cristallisation ;
- une étape de liquéfaction du méthane récupérée, au moyen de l'échangeur de liquéfaction ;
- une étape de récupération de méthane liquide en sortie de l'échangeur de liquéfaction ; et
- une étape de renvoi d'une partie du méthane liquide vers le système de cristallisation, ladite partie du méthane liquide formant le flux comprenant en majorité du méthane liquide.

Selon le cas, le procédé selon l'invention peut présenter une ou plusieurs des caractéristiques ci-dessous :
- le flux gazeux d'alimentation comprend de l'eau et ledit procédé comprend avant l'étape de cristallisation : une étape d'abattement de la concentration en eau comprise dans le flux gazeux d'alimentation par abaissement de la température du flux gazeux d'alimentation à une température comprise entre - 57°C et -75°C ; et une étape de récupération d'un flux gazeux d'alimentation appauvri en eau ;
- l'étape d'abattement de la concentration en eau comprend un abaissement de la température du flux gazeux d'alimentation en deux temps un premier temps durant lequel la température du flux gazeux d'alimentation est abaissée à une température comprise entre 0 °C et 10°C, et un deuxième temps durant lequel la température du flux gazeux d'alimentation est abaissée à une température comprise entre - 57°C et - 75°C;
- pendant l'étape de cristallisation le ratio du débit du flux gazeux d'alimentation et du débit du flux de méthane liquide à contre-courant est tel que les particules formées de CO2 solide sont entraînées vers le fond inférieur du système de cristallisation ;
- après l'étape de cristallisation : on récupère au fond inférieur du système de cristallisation un mélange de méthane liquide et de particules de CO2 solide, on sépare les phases liquide et solide dudit mélange de manière à récupérer les particules de CO2 solide, et on récupère en sortie de la séparation des phases un flux présentant une teneur en dioxyde de carbone comprise entre 2 ppm et 1000 ppm, de préférence entre 5 et 200 ppm ;
- le flux présentant une teneur en dioxyde de carbone comprise entre 2 ppm et 1000 ppm, de préférence entre 5 et 200 ppm est recyclé dans le système de cristallisation et/ou dans l'échangeur de liquéfaction ;
- les particules de CO2 solide sont utilisées pour apporter du froid à un cycle de refroidissement de l'échangeur de liquéfaction ;
- le flux gazeux d'alimentation est du biogaz ;
- le flux gazeux d'alimentation est à une pression comprise entre la pression atmosphérique et 20 bar, de préférence entre la pression atmosphérique et 5 bar.

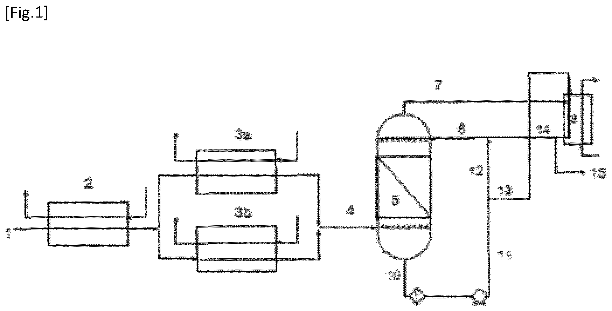

L'invention va à présent être décrite plus en détail à l'aide de la figure 1 [Fig. 1].

L'eau du flux gazeux d'alimentation est tout d'abord condensée. Pour ce faire, le flux gazeux d'alimentation 1 est dans un premier temps, amené à une température comprise entre 0 et 10°C (1°C par exemple), au moyen d'un échangeur 2, afin de condenser une partie de l'eau contenue dans le biogaz et de l'éliminer à l'état liquide. Cette étape permet de réduire la quantité de solide à éliminer dans les échangeurs en aval.

Dans un deuxième temps, le biogaz est amené à une température comprise entre -57°C et - 75°C afin d'éliminer la majorité d'eau présente et les impuretés ou contaminants de type COV présents dans le flux gazeux d'alimentation. Deux échangeurs 3a et 3b sont placés en parallèle pour refroidir le biogaz jusqu'à cette température. Les échangeurs sont régénérés une fois le solide déposé sur les parois. Le temps de cycle de chaque échangeur comprenant une phase de production et une phase de régénération comprise entre 10 min et 10 h de préférence comprise entre 30 min et 2h.

Dans le cadre de l'invention, Le premier temps et le deuxième temps sont regroupés en une étape appelée « abattement de la concentration en eau ».

On récupère en sortie des échangeurs un flux gazeux 4 appauvri en eau et en impuretés. Ce flux gazeux 4 est ensuite injecté dans un système de cristallisation 5 à contre-courant d'un mélange 6 comprenant principalement du méthane liquide. Le gaz en contact avec le liquide va provoquer la cristallisation du CO2 contenu dans le gaz au fur et à mesure de son refroidissement (refroidissement entre -50°C et -163 °C). Notons que l'on pourra observer en même temps mais dans une moindre mesure la cristallisation de l'H₂S contenu dans le flux d'alimentation.

Le ratio du débit du flux gazeux d'alimentation et du débit du flux de méthane liquide à contre-courant est tel que l'on assure l'entraînement des particules de CO2 solide vers le fond inférieur du système de cristallisation, et que le gaz en haut du système de cristallisation ne contient pratiquement pas de CO2. Le débit de liquide en excès, assure notamment un très bon mouillage de toute la surface présente à l'intérieur du système de cristallisation et utilisée comme « contacteur » entre le liquide et le gaz. A titre d'exemple on pourra utiliser comme contacteur les garnissages structurés.

On récupère ainsi du méthane gazeux 7 en partie supérieure du système de cristallisation qui est envoyé vers un échangeur de liquéfaction 8 de manière à produire du méthane liquide. Une partie 14 de ce méthane liquide sera renvoyée vers le système de cristallisation et une autre partie 15 de ce méthane liquide sera envoyée vers la production de méthane liquide.

A la sortie du fond inférieur du système de cristallisation, on récupère un mélange 10 de méthane liquide et de particules de CO2 solide. On sépare les phases dudit mélange de manière à récupérer les particules de CO2 solide, et on récupère en sortie de la séparation un flux de méthane liquide 11 comprenant entre 2 et 1000 ppm de CO2, de préférence entre 5 et 200 ppm de CO2. Notons que la séparation de phases pourra être réalisée au moyen d'un filtre ou de plusieurs filtres en parallèle et en fonctionnement alterné, ou toute autre système de séparation liquide solide.

Le flux de méthane liquide 11 récupéré en sortie de la filtration est d'une part recyclé 12 vers la tête du système de cristallisation, et d'autre part envoyé 13 vers l'échangeur de liquéfaction du méthane pour assurer un mouillage de la surface d'échange et éviter les dépôts de cristaux de CO2 dans l'échangeur de liquéfaction.

Le procédé selon l'invention nécessite un apport de puissance frigorifique pour fonctionner. Cet apport peut se réaliser de plusieurs manières (en fonction de la quantité de biométhane liquide à produire par exemple). A titre d'exemple, mais pas exclusivement : 1. A partir d'une source d'azote liquide 2. Par un procédé type Brayton inversé: Dans ce dernier cas un fluide frigorigène (de l'azote, ou un mélange Azote - Helium est comprimé, refroidi et détendu dans une turbine. Ce fluide est ensuite réchauffé à contre courant des fluides chauds (y compris le biogaz) dans les échangeurs. Il apporte le froid nécessaire à son refroidissement jusqu'à -75°C d'une part, et à la liquéfaction de la vapeur de méthane qui sort du système de cristallisation). D'un point de vue thermodynamique, lorsque le mélange principalement composé de CO2 et de méthane est refroidi, le CO2 commence à se solidifier en dessous d'un certain seuil de température depuis la phase gaz (passage directement de CO2 vapeur à CO2 solide). La température d'apparition des premiers cristaux de CO2 solide est estimée à -87°C environ pour 1 bara et 50% molaire de CO2 (i.e. température inférieure à la température de sortie des 2 échangeurs en parallèle). En sortie liquide du système de cristallisation, le CO2 solide et la phase liquide sont proches de l'équilibre thermodynamique. Le solide est alors filtré en amont de la pompe pour ne garder que la phase liquide. Néanmoins, une faible quantité de CO2 solide pourra se former à nouveau en présence de méthane liquide dans l'échangeur de reflux liquide. On pourra alors mettre si besoin soit 2 échangeurs en parallèle (un en production et un autre en régénération), soit un système de séparation de phases solide-liquide sur la ligne de biométhane liquide produit.

Notons que le CO2 solide récupéré dans le système de séparation de phases, peut être utilisé pour apporter du froid au cycle de refroidissement et réduire ainsi la consommation spécifique du cycle de cryo-solidification.

Notons que le procédé décrit est conçu pour fonctionner à basse pression entre la pression atmosphérique et 20 bar, de préférence entre la pression atmosphérique et 5 bar.

## Revendications

1. Installation de production de méthane liquide à partir d'un flux gazeux d'alimentation (1) comprenant au moins du méthane et du dioxyde de carbone comprenant dans le sens de circulation du flux gazeux :
- un système de cristallisation (5) du dioxyde de carbone fonctionnant en continu comprenant à contre-courant du flux gazeux d'alimentation comprenant au moins du méthane et du dioxyde de carbone la circulation d'un flux (6) comprenant en majorité du méthane liquide, et permettant la production un flux gazeux enrichi en méthane (7),
- un échangeur de liquéfaction (8) du flux gazeux enrichi en méthane sortant du système de cristallisation, permettant de produire du méthane liquide,
- un moyen permettant de récupérer le méthane liquide (14, 15) en sortie de l'échangeur de liquéfaction (8), et
- un moyen permettant de renvoyer une partie (14) du méthane liquide vers le système de cristallisation (5), ladite partie (14) du méthane liquide formant le flux (6) comprenant en majorité du méthane liquide.

2. Installation selon la revendication 1, **caractérisée en ce que** ladite installation comprend en amont du système de cristallisation (5) un moyen C permettant d'amener le flux gazeux d'alimentation à une température comprise entre -50°C et - 85°C, de préférence entre -57°C et -75°C.

3. Installation selon la revendication 2, **caractérisée en ce que** le flux gazeux d'alimentation comprend de l'eau et le moyen C comprend au moins deux échangeurs disposés en parallèle (3a) et (3b), les échangeurs suivant chacun un cycle comprenant une étape de production et une étape de régénération, avec à chaque instant du cycle un échangeur en étape de production et un échangeur en régénération.

4. Installation selon l'une des revendications 2 ou 3, **caractérisée en ce qu'**elle comprend en amont du moyen C un moyen C' (2) permettant d'amener le flux gazeux d'alimentation à une température comprise entre 0°C et 20°C, de préférence comprise entre 0°C et 10°C.

5. Installation selon l'une des revendications 1 à 4, **caractérisée en ce qu'**elle comprend :
- un conduit relié à un fond inférieur du système de cristallisation (5), et
- un système de séparation de phases liquide-solide placé sur ce conduit et permettant de récupérer des cristaux de CO2.

6. Installation selon l'une des revendications 1 à 5, **caractérisée en ce que** le flux d'alimentation est à une pression comprise entre la pression atmosphérique et 20 bar, de préférence entre la pression atmosphérique et 5 bar.

7. Procédé de production de méthane liquide à partir d'un flux gazeux d'alimentation (1) comprenant au moins du méthane et du dioxyde de carbone et mettant en oeuvre une installation telle que définie dans l'une des revendications 1 à 6, ledit procédé comprenant :
- une étape continue de cristallisation du dioxyde de carbone contenu dans le flux gazeux d'alimentation comprenant au moins du méthane et du dioxyde de carbone par injection de celui-ci dans le système de cristallisation (5) du dioxyde de carbone comprenant à contre-courant du flux gazeux d'alimentation la circulation d'un flux (6) comprenant en majorité du méthane liquide ;
- une étape de récupération de méthane gazeux (7) en partie supérieure du système de cristallisation (5);
- une étape de liquéfaction du méthane récupérée, au moyen de l'échangeur de liquéfaction (8) ;
- une étape de récupération de méthane liquide (14, 15) en sortie de l'échangeur de liquéfaction (8); et
- une étape de renvoi d'une partie (14) du méthane liquide vers le système de cristallisation (5), ladite partie du méthane liquide formant le flux (6) comprenant en majorité du méthane liquide.

8. Procédé selon la revendication 7, **caractérisé en ce que** le flux gazeux d'alimentation comprend de l'eau et ledit procédé comprend avant l'étape de cristallisation :
- une étape d'abattement de la concentration en eau comprise dans le flux gazeux d'alimentation par abaissement de la température du flux gazeux d'alimentation à une température comprise entre -57°C et -75°C ; et
- une étape de récupération d'un flux gazeux d'alimentation (4) appauvri en eau.

9. Procédé selon la revendication 8, **caractérisé en ce que** l'étape d'abattement de la concentration en eau comprend un abaissement de la température du flux gazeux d'alimentation en deux temps :
- un premier temps durant lequel la température du flux gazeux d'alimentation est abaissée à une température comprise entre 0 °C et 10°C, et
- un deuxième temps durant lequel la température du flux gazeux d'alimentation est abaissée à une température comprise entre -57°C et -75°C.

10. Procédé selon l'une des revendications 7 à 9, **caractérisé en ce que** pendant l'étape de cristallisation le ratio du débit du flux gazeux d'alimentation et du débit du flux de méthane liquide à contre-courant est tel que les particules formées de CO2 solide sont entraînées vers un fond inférieur du système de cristallisation (5).

11. Procédé selon la revendication 9, **caractérisé en ce qu'**après l'étape de cristallisation:
- on récupère au fond inférieur du système de cristallisation (5) un mélange (10) de méthane liquide et de particules de CO2 solide,
- on sépare les phases liquide et solide dudit mélange de manière à récupérer les particules de CO2 solide, et
- on récupère en sortie de la séparation des phases un flux de méthane liquide (11) présentant une teneur en dioxyde de carbone comprise entre 2 ppm et 1000 ppm, de préférence entre 5 et 200 ppm.

12. Procédé selon la revendication 11, **caractérisé en ce que** le flux de méthane liquide comprenant entre 2 et 1000 ppm de CO2, de préférence entre de 5 ppm et 200 ppm de CO2 est recyclé (12, 13) dans le système de cristallisation (5) et/ou dans l'échangeur de liquéfaction (8).

13. Procédé selon l'une des revendications 11 ou 12, **caractérisé en ce que** les particules de CO2 solide sont utilisées pour apporter du froid à un cycle de refroidissement de l'échangeur de liquéfaction (8).

14. Procédé selon l'une des revendications 7 à 13, **caractérisé en ce que** le flux gazeux d'alimentation est du biogaz.

15. Procédé selon l'une des revendications 7 à 14, **caractérisé en ce que** le flux gazeux d'alimentation est à une pression comprise entre la pression atmosphérique et 20 bar, de préférence entre la pression atmosphérique et 5 bar.

## Patentansprüche

1. Anlage zur Erzeugung von flüssigem Methan aus einem zumindest Methan und Kohlendioxid umfassenden zugeführten Gasstrom (1), die in der Strömungsrichtung des Gasstroms Folgendes umfasst:
- ein kontinuierlich betriebenes System (5) zur Kristallisation von Kohlendioxid, das im Gegenstrom zum zumindest Methan und Kohlendioxid umfassenden zugeführten Gasstrom den Umlauf eines überwiegend flüssiges Methan umfassenden Stroms (6) umfasst und die Erzeugung eines mit Methan angereicherten Gasstroms (7) ermöglicht,
- einen Austauscher (8) zur Verflüssigung des mit Methan angereicherten Gasstroms, der aus dem Kristallisationssystem austritt, wodurch die Erzeugung von flüssigem Methan ermöglicht wird,
- ein Mittel, welches das Gewinnen des flüssigen Methans (14, 15) am Auslass des Verflüssigungsaustauschers (8) ermöglicht, und
- ein Mittel, welches das Rückführen eines Teils (14) des flüssigen Methans zum Kristallisationssystem (5) ermöglicht, wobei der Teil (14) des flüssigen Methans, der den Strom (6) bildet, überwiegend flüssiges Methan umfasst.

2. Anlage nach Anspruch 1, **dadurch gekennzeichnet, dass** die Anlage stromaufwärts vom Kristallisationssystem (5) ein Mittel C umfasst, welches das Bringen des zugeführten Gasstroms auf eine Temperatur zwischen -50 °C und -85 °C, vorzugsweise zwischen -57 °C und -75 °C, ermöglicht.

3. Anlage nach Anspruch 2, **dadurch gekennzeichnet, dass** der zugeführte Gasstrom Wasser umfasst und das Mittel C mindestens zwei parallel angeordnete Austauscher (3a) und (3b) umfasst, wobei die Austauscher jeweils einem Zyklus folgen, der einen Erzeugungsschritt und einen Regenerationsschritt umfasst, wobei sich zu jedem Zeitpunkt des Zyklus ein Austauscher im Erzeugungsschritt und ein Austauscher im Regenerationsschritt befindet.

4. Anlage nach einem der Ansprüche 2 oder 3, **dadurch gekennzeichnet, dass** sie stromaufwärts vom Mittel C ein Mittel C' (2) umfasst, welches das Bringen des zugeführten Gasstroms auf eine Temperatur zwischen 0 °C und 20 °C, vorzugsweise zwischen 0 °C und 10 °C, ermöglicht.

5. Anlage nach einem der Ansprüche 1 bis 4, **dadurch gekennzeichnet, dass** sie Folgendes umfasst:
- eine Leitung, die mit einem unteren Boden des Kristallisationssystems (5) verbunden ist, und
- ein System zur Flüssig-Fest-Phasentrennung, das an dieser Leitung angeordnet ist und das Gewinnen von CO2-Kristallen ermöglicht.

6. Anlage nach einem der Ansprüche 1 bis 5, **dadurch gekennzeichnet, dass** der Zufuhrstrom einen Druck zwischen dem Atmosphärendruck und 20 bar, vorzugsweise zwischen dem Atmosphärendruck und 5 bar, aufweist.

7. Verfahren zur Erzeugung von flüssigem Methan aus einem zumindest Methan und Kohlendioxid umfassenden zugeführten Gasstrom (1) und wobei eine Anlage gemäß der Definition in einem der Ansprüche 1 bis 6 verwendet wird, wobei das Verfahren Folgendes umfasst:
- einen kontinuierlichen Schritt zur Kristallisation von Kohlendioxid, das in dem zumindest Methan und Kohlendioxid umfassenden zugeführten Gasstrom enthalten ist, durch dessen Einspritzen in das System (5) zur Kristallisation von Kohlendioxid, das im Gegenstrom zum zugeführten Gasstrom den Umlauf eines überwiegend flüssiges Methan umfassenden Stroms (6) umfasst;
- einen Schritt zur Gewinnung von gasförmigem Methan (7) im oberen Teil des Kristallisationssystems (5);
- einen Schritt des Verflüssigens des gewonnenen Methans mittels des Verflüssigungsaustauschers (8);
- einen Schritt des Gewinnens von flüssigem Methan (14, 15) am Auslass des Verflüssigungsaustauschers (8) und
- einen Schritt des Rückführens eines Teils (14) des flüssigen Methans zum Kristallisationssystem (5), wobei der Teil des flüssigen Methans, der den Strom (6) bildet, überwiegend flüssiges Methan umfasst.

8. Verfahren nach Anspruch 7, **dadurch gekennzeichnet, dass** der zugeführte Gasstrom Wasser umfasst und das Verfahren vor dem Kristallisationsschritt Folgendes umfasst:
- einen Schritt des Verminderns der Konzentration von im zugeführten Gasstrom enthaltenem Wasser durch eine Erniedrigung der Temperatur des zugeführten Gasstroms auf eine Temperatur zwischen -57 °C und -75 °C und
- einen Schritt des Gewinnens eines an Wasser verarmten zugeführten Gasstroms (4).

9. Verfahren nach Anspruch 8, **dadurch gekennzeichnet, dass** der Schritt der Verminderung der Wasserkonzentration eine Erniedrigung der Temperatur des zugeführten Gasstroms in zwei Zeitabschnitten umfasst:
- einem ersten Zeitabschnitt, in dem die Temperatur des zugeführten Gasstroms auf eine Temperatur zwischen 0 °C und 10 °C erniedrigt wird, und
- einem zweiten Zeitabschnitt, in dem die Temperatur des zugeführten Gasstroms auf eine Temperatur zwischen - 57 °C und -75 °C erniedrigt wird.

10. Verfahren nach einem der Ansprüche 7 bis 9, **dadurch gekennzeichnet, dass** beim Kristallisationsschritt das Verhältnis des Durchsatzes des zugeführten Gasstroms zum Durchsatz des flüssigen Methans im Gegenstrom so ist, dass die gebildeten festen CO2-Partikel in Richtung eines unteren Bodens des Kristallisationssystems (5) mitgerissen werden.

11. Verfahren nach Anspruch 9, **dadurch gekennzeichnet, dass** nach dem Kristallisationsschritt:
- am unteren Boden des Kristallisationssystems (5) eine Mischung (10) aus flüssigem Methan und festen CO2-Partikeln gewonnen wird,
- die flüssige und die feste Phase der Mischung getrennt wird, um die festen CO2-Partikel zu gewinnen, und
- am Auslass der Phasentrennung ein flüssiger Methanstrom (11) gewonnen wird, der einen Kohlendioxidgehalt zwischen 2 ppm und 1000 ppm, vorzugsweise zwischen 5 und 200 ppm, aufweist.

12. Verfahren nach Anspruch 11, **dadurch gekennzeichnet, dass** der flüssige Methanstrom, der zwischen 2 und 1000 ppm CO2, vorzugsweise zwischen 5 ppm und 200 ppm CO2, umfasst, in das Kristallisationssystem (5) und/oder in den Verflüssigungsaustauscher (8) zurückgeführt (12, 13) wird.

13. Verfahren nach einem der Ansprüche 11 oder 12, **dadurch gekennzeichnet, dass** die festen CO2-Partikel verwendet werden, um Kälte in einen Kühlzyklus des Verflüssigungsaustauschers (8) einzubringen.

14. Verfahren nach einem der Ansprüche 7 bis 13, **dadurch gekennzeichnet, dass** es sich beim zugeführten Gasstrom um Biogas handelt.

15. Verfahren nach einem der Ansprüche 7 bis 14, **dadurch gekennzeichnet, dass** der zugeführte Gasstrom einen Druck zwischen dem Atmosphärendruck und 20 bar, vorzugsweise zwischen dem Atmosphärendruck und 5 bar, aufweist.

## Claims

1. Plant for the production of liquid methane from a feed gas stream (1) comprising at least methane and carbon dioxide comprising, in the direction of circulation of the gas stream:
- a continuously operating system (5) for the crystallization of carbon dioxide comprising, countercurrentwise to the feed gas stream comprising at least methane and carbon dioxide, the circulation of a stream (6) predominantly comprising liquid methane, and making possible the production of a methane-enriched gas stream (7),
- an exchanger (8) for liquefaction of the methane-enriched gas stream exiting from the crystallization system, making it possible to produce liquid methane,
- a means making it possible to recover the liquid methane (14, 15) at the outlet of the liquefaction exchanger (8), and
- a means making it possible to return a part (14) of the liquid methane to the crystallization system (5), said part (14) of the liquid methane forming the stream (6) predominantly comprising liquid methane.

2. Plant according to Claim 1, **characterized in that** said plant comprises, upstream of the crystallization system (5), a means C which makes it possible to bring the feed gas stream to a temperature of between -50°C and -85°C, preferably between -57°C and -75°C.

3. Plant according to Claim 2, **characterized in that** the feed gas stream comprises water and the means C comprises at least two exchangers (3a) and (3b) arranged in parallel, the exchangers each following a cycle comprising a production stage and a regeneration stage, with, at each moment of the cycle, an exchanger in the production stage and an exchanger in the regeneration stage.

4. Plant according to either of Claims 2 and 3, **characterized in that** it comprises, upstream of the means C, a means C' (2) which makes it possible to bring the feed gas stream to a temperature of between 0°C and 20°C, preferably of between 0°C and 10°C.

5. Plant according to one of Claims 1 to 4, **characterized in that** it comprises:
- a pipe connected to a lower bottom of the crystallization system (5), and
- a system for separation of liquid/solid phases placed on this pipe and making it possible to recover CO₂ crystals.

6. Plant according to one of Claims 1 to 5, **characterized in that** the feed stream is at a pressure of between atmospheric pressure and 20 bar, preferably between atmospheric pressure and 5 bar.

7. Process for the production of liquid methane from a feed gas stream (1) comprising at least methane and carbon dioxide which employs a plant as defined in one of Claims 1 to 6, said process comprising:
- a continuous stage of crystallization of the carbon dioxide contained in the feed gas stream comprising at least methane and carbon dioxide by injection of this into the system (5) for the crystallization of carbon dioxide comprising, countercurrentwise to the feed gas stream, the circulation of a stream (6) predominantly comprising liquid methane;
- a stage of recovery of gaseous methane (7) in the upper part of the crystallization system (5);
- a stage of liquefaction of the recovered methane, by means of the liquefaction exchanger (8);
- a stage of recovery of liquid methane (14, 15) at the outlet of the liquefaction exchanger (8); and
- a stage of returning a part (14) of the liquid methane to the crystallization system (5), said part of the liquid methane forming the stream (6) predominantly comprising liquid methane.

8. Process according to Claim 7, **characterized in that** the feed gas stream comprises water and said process comprises, before the crystallization stage:
- a stage of reducing the concentration of water included in the feed gas stream by lowering the temperature of the feed gas stream to a temperature of between -57°C and -75°C; and
- a stage of recovery of a feed gas stream (4) depleted in water.

9. Process according to Claim 8, **characterized in that** the stage of reducing the concentration of water comprises a lowering of the temperature of the feed gas stream in two steps:
- a first step during which the temperature of the feed gas stream is lowered to a temperature of between 0°C and 10°C, and
- a second step during which the temperature of the feed gas stream is lowered to a temperature of between - 57°C and -75°C.

10. Process according to one of Claims 7 to 9, **characterized in that**, during the crystallization stage, the ratio of the flow rate of the feed gas stream to the flow rate of the countercurrentwise liquid methane stream is such that the solid CO₂ particles formed are carried towards a lower bottom of the crystallization system (5).

11. The process according to Claim 9, **characterized in that**, after the crystallization stage:
- a mixture (10) of liquid methane and of solid CO₂ particles is recovered at the lower bottom of the crystallization system (5),
- the liquid and solid phases of said mixture are separated so as to recover the solid CO₂ particles, and
- a stream of liquid methane (11) exhibiting a carbon dioxide content of between 2 ppm and 1000 ppm, preferably between 5 ppm and 200 ppm, is recovered at the outlet of the separation of the phases.

12. Process according to Claim 11, **characterized in that** the liquid methane stream comprising between 2 ppm and 1000 ppm of CO₂, preferably between 5 ppm and 200 ppm of CO₂, is recycled (12, 13) in the crystallization system (5) and/or in the liquefaction exchanger (8).

13. Process according to either of Claims 11 and 12, **characterized in that** the solid CO₂ particles are used to contribute cold to a cooling cycle of the liquefaction exchanger (8).

14. Process according to one of Claims 7 to 13, **characterized in that** the feed gas stream is biogas.

15. Process according to one of Claims 7 to 14, **characterized in that** the feed gas stream is at a pressure of between atmospheric pressure and 20 bar, preferably between atmospheric pressure and 5 bar.
